(19) Europäisches Patentamt

European Patent Office

Office européen des brevets

(11) **EP 0 586 592 B1**

(12) **EUROPEAN PATENT SPECIFICATION**

(45) Date of publication and mention
of the grant of the patent:
**29.11.2000 Bulletin 2000/48**

(51) Int. Cl.[7]: **A61K 38/28**

(21) Application number: **92913644.8**

(22) Date of filing: **23.05.1992**

(86) International application number:
**PCT/US92/04351**

(87) International publication number:
**WO 92/20366 (26.11.1992 Gazette 1992/29)**

(54) **INSULIN AND AMYLIN CONTAINING COMPOSITION FOR THE TREATMENT OF INSULIN DEFICIENT MAMMALS**

Zusammensetzung zur Behandlung des Insulinmangels bei Säugetieren, die Amylin und Insulin enthält.

TRAITEMENT DES MAMMIFERES SOUFFRANT D'UNE CARENCE INSULINIQUE

(84) Designated Contracting States:
**AT BE CH DE DK ES FR GB GR IT LI LU MC NL SE**

(30) Priority: **24.05.1991 US 704995**

(43) Date of publication of application:
**16.03.1994 Bulletin 1994/11**

(73) Proprietor:
**AMYLIN PHARMACEUTICALS, INC.
San Diego, CA 92121 (US)**

(72) Inventors:
• **RINK, Timothy, J.
La Jolla, CA 92037 (US)**
• **YOUNG, Andrew, A.
San Diego, CA 92121 (US)**

(74) Representative:
**Goldin, Douglas Michael et al
J.A. KEMP & CO.
14 South Square
Gray's Inn
London WC1R 5LX (GB)**

(56) References cited:
**EP-A- 0 309 100          EP-A- 0 408 284**

**Description**

Field of the Invention

This invention relates to therapeutic combination of insulin and amylin and their use in the manufacture of medicaments for treatment of insulin deficient mammals, such as diabetic humans.

Background of the Invention

Human diabetics are deficient in insulin secretion, and in some cases lack insulin. Insulin is one of several hormones which play a role in regulation of blood glucose levels. Simplistically, there are two main stores of glucose in a mammal--the liver and skeletal muscle, where glucose is stored in the form of glycogen. Muscle glycogen is used as a glucose source for the muscle, whereas liver glycogen is used as a glucose source for all tissues, including blood. It is the interplay of certain hormones in regulation of glycogen accumulation and breakdown that is critical in the invention described below.

Insulin regulates glucose uptake by muscle tissue for storage of the glucose as muscle glycogen. Insulin also prevents hyperglycemia, that is, the unacceptable accumulation of high levels of glucose in the blood, and suppresses conversion of liver glycogen to glucose, and subsequent secretion of that glucose into the blood. In the presence of excess insulin, blood glucose accumulates in muscle tissue as glycogen, liver glucose output is suppressed, and the level of blood glucose falls, to create a condition termed hypoglycemia.

Another hormone, glucagon, increases blood glucose levels by stimulating liver glycogen breakdown to glucose, and subsequent secretion of that glucose. This liver glycogen is used to maintain blood glucose levels, and glucagon may be considered an insulin counterregulatory hormone.

Amylin is another hormone which has been discovered to be concerned in regulation of blood glucose levels. It reverses insulin-mediated suppression of liver glucose output in rats. Molina et al., 39 <u>Diabetes</u> 260, 1990, and Koopmans et al., 39 <u>Diabetes</u> 101A, 1990.

European Patent Application No. 88307927.9 (EP-A-0,309,100) describes the treatment of diabetes mellitus or hypoglycemia with amylin, or with a combination of amylin and insulin, preferably at a ratio of between 100:1 to 0.1:1 insulin to amylin. The applicant reports that the major problem with insulin treatment of diabetes is hypoglycemia and that co-administration of insulin and amylin will avoid this side effect or otherwise reduce its risk and/or severity. The application states that amylin exerts a powerful modulating effect on insulin-induced storage of glucose as glycogen, and that amylin will act to modulate and reduce the hypoglycemic effects of insulin by reducing release of insulin in relation to a given glucose stimulus, and by reducing the rate of storage of glucose as muscle glycogen. That is, amylin should reduce insulin-sensitivity and cushion the hypoglycemic effects of insulin.

Summary of the Invention

Applicants' invention is concerned with discovery of a further surprising relationship between amylin and insulin. It also relates to the unexpected discovery of optimum ratios of insulin and amylin used for treatment of mammals suffering from diabetes, or other insulin deficient mammals. Applicant has discovered that it is preferable to maintain a ratio of amylin and insulin within a mammal in a relatively narrow range or ranges in order that both the amylin and insulin will have optimal effects, and prevent the damaging side effects of either excess amylin or insulin alone.

In view of amylin's antagonism of muscle response to insulin, others have postulated that the effects of amylin and insulin would simply cancel each other out <u>in vivo</u>. However, applicants discovered that not only is this not the case, but that amylin suppression of the insulin response of a patient is increased as the hormonal concentrations increase above a certain value. Thus, when the hormones are provided in a fixed ratio, the effects of each hormone are moderated, and the damaging effect of either hormone alone at high concentration is prevented.

Amylin is a functional non-competitive antagonist of insulin in regard to muscle glycogen metabolism, for example. That is, regardless of insulin concentration and the level of insulin-stimulation, amylin at appropriate concentrations will nonetheless diminish the insulin response. When amylin and insulin are present in a suitable fixed ratio, increasing concentrations of the mixture have the initial effect of insulin stimulation, and increases in muscle glycogen synthesis, for example, occur. With further increases of concentration of the mixture, the response to the insulin component increases little further but the non-competitive antagonism of the amylin component continues. The net response to further increases of the mixture is in fact diminished, resulting in a bell-shaped dose-response curve, rather than the sigmoid response typically obtained with insulin alone.

Applicants discovered that provision of amylin and insulin at a constant molar ratio results in a bell-shaped dose response relationship between hormone levels and glucose disposal in skeletal muscle indicating that amylin and insulin act independently of one another, and are non-competitive functional antagonists for certain key metabolic responses, including glucose uptake into skeletal muscle glycogen and hepatic glucose output. Applicants propose in this invention that patients suffering from diabetes may be treated in a manner, for example, which maintains the insulin and amylin within the patient at relatively constant proportions, including those which may be similar to those present in normal mammals under physiologic conditions. This is advantageous because, at normal physiologic insulin and amylin concentrations, the response of the body to insulin is not much altered in the presence of amylin, but at high insulin and amylin levels the response will be considerably lower than maximum in the presence of amylin. Thus, insulin mediated side-effects are suppressed by amylin at high insulin concentrations. Administration of insulin and amylin in a given proportion or the provision of at least about 0.5 mg/kg/ day amylin, reduces the risk of catastrophic hypoglycemia that would otherwise follow the introduction of excessive levels of insulin. In the regime proposed, the patient is protected from an overdose of insulin since amylin dampens potential oscillations of plasma-glucose level caused by poor insulin regulation. Without being bound to any theory, applicants believe that the coadministered amylin operates by restoring hepatic glycogen stores so that, in the presence of insulin, such glycogen is mobilized by glucagon.

One aim of coadminstration of amylin and insulin in diabetes is to restore, as far as practicable, the most effective ratio of these hormones. Thus, the dose of each will reflect their respective deficit due to beta cell loss, and will be related approximately in the ratio of amylin to insulin normally co-secreted by a pancreatic beta cell. Applicants have discovered that a molar ratio of between about 3% and-about 40% amylin to insulin is indicated, i.e., an amylin to insulin ratio of about 1:35 to about 1:2.5. This may vary for different patients and the approximate ratio will be determined by (1) monitoring residual amylin and insulin levels by plasma immunoassay, and (2) by normal practice clinical monitoring of plasma glucose and clinical state.

It is desirable to provide relatively constant amylin:insulin ratios, particularly when insulin levels are likely to be changing. It is not likely, however, that a depot form of amylin, to match, say, ultralente insulin will be required. Ultralente provides a rather steady basal insulin level, not predicted to elicit hypoglycemic episodes. The main need is for effective levels of amylin to accompany and track insulin levels following administration of faster acting forms of insulin that lead to rapidly changing plasma levels.

Thus, amylin should be formulated to provide a pattern of plasma concentrations which matches, within a factor of about 2.5-fold, in time-to-peak and half-time for disappearance, that of the soluble or rapid-acting insulin being administered to a patient. In this way, amylin will be most moderating of insulin action when insulin is the higher concentration range, and will leave insulin action essentially intact as plasma levels due to soluble insulin decline. The amylin administered will not significantly impact the ability of the ultralente contribution of basal insulin to limit upswings in plasma glucose.

In summary, the preferred formulation for amylin is one which provides amylin/insulin concentration ratios within about 33% to about 300% of those in normal subjects for 4 hours following administration of the patient's most rapid acting insulin dosage, and matches, within a factor of about 3-fold, the time-to-peak and the time course of decline of plasma insulin levels.

Thus, in a first aspect, the invention provides a composition comprising an insulin having an *in vitro* activity of stimulating glucose incorporation into glycogen in rat soleus muscle, and an amylin having the *in vitro* activity of suppressing glucose incorporation into glycogen in rat soleus muscle, wherein said insulin and amylin are provided in a molar ratio of between 1:1 and 40:1.

In a second aspect the invention provides the use of a composition comprising an insulin and an amylin wherein said insulin and amylin are provided in a molar ratio of between 1:1 and 40:1 for the manufacture of a medicament for the treatment of a mammal having a reduced ability to produce serum insulin compared to a normal mammal.

By "an insulin" is meant any insulin of natural or synthetic origin, and functional peptide fragments and conservative variants of an insulin which may be used in conventional treatment of diabetes mellitus. It is important in this invention, and it is clear to those of ordinary skill in this art, that the insulin polypeptide fragment need only have an insulin activity characterized by the in vitro activity of stimulating glucose incorporation into glycogen in rat soleus muscle, as described in detail below. Thus, the insulin may be produced in any standard manner including, but not limited to, extraction of bovine and porcine pancreatic insulin, expression of the insulin from recombinant DNA, and in vitro polypeptide synthesis of fragments of insulin. The insulin may also be provided in a fast-acting or semilente form.

By conservative variant is meant an insulin polypeptide which has substantial (at least 90% of a contiguous ten amino acid sequence) amino acid sequence identity with a naturally occurring insulin, or one used in conventional treatment of diabetes mellitus, but which has one or more amino acids in such a sequence substituted in a conservative manner, such that the biological activity of the insulin remains substantially intact. Examples of such a conservative substitution includes substituting positively charged amino acids for other positively charged amino acids, negatively charged amino acids for other negatively charged amino acids, and simple amino acids, such as glycine, for other such amino acids, such as valine. Useful insulins for this invention can be readily determined by testing a chosen insulin for the desired activity in the in vitro test described below, or any equivalent in vitro or in vivo test.

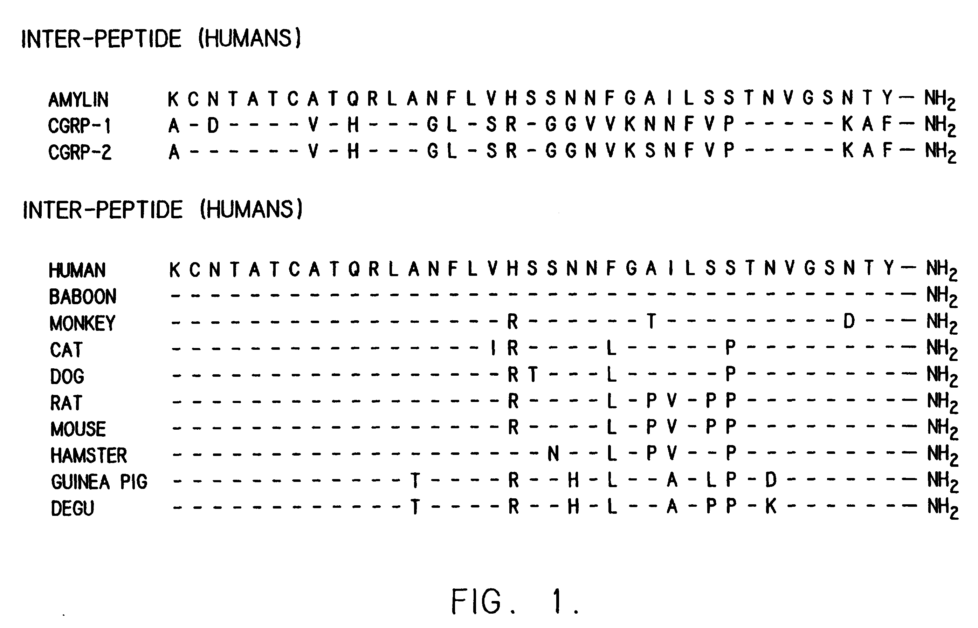

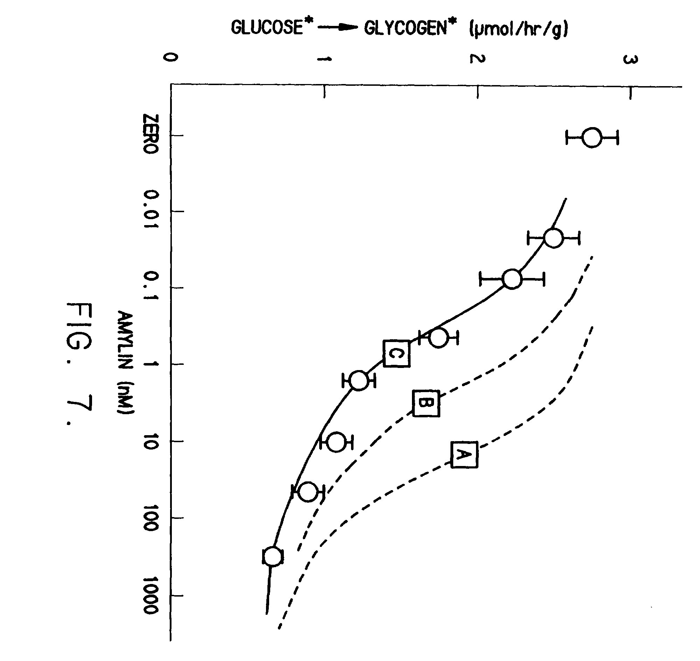

By "an amylin" is meant any agonist molecule which includes a functional peptide fragment of a naturally occurring amylin, carboxy-deamidated amylin or calcitonin gene related peptide (CGRP) which includes at least five

EP 0 586 592 B1

amino acid residues and has the in vitro activity described above. In addition, it is preferred that such an amylin perform in vivo a therapeutic function of the complete amylin or CGRP peptide. The term also includes conservative variants and functional peptide fragments of such peptides. Examples of such amylins are provided in Fig. 1 where sequence homologies among amylins in humans and other species are shown (using a standard one letter amino acid code). Preferably, the amylin has a Kd of less than 60 pMolar in a receptor assay, and an $EC_{50}$ of less than 1nMolar in a rat soleus assay (Fig. 7, see below).

Those of ordinary skill in this art will recognize that the above terms "insulin" and "amylin" can be read broadly to include any polypeptide or other chemical class having the above described desired biological activity, *in vitro* or *in vivo*, which stimulates or suppresses, respectively, glucose incorporation into glycogen in any of many test systems, including, rat soleus muscle. In addition, such persons recognize that the polypeptide may be provided in a carboxy-terminated form, carboxy-terminally amidated form, or any other form which does not significantly affect the desired biological activity of the polypeptide. For example, as described in EP-A-0,309,100, *supra*, the amylin may be prepared in a soluble form.

In preferred embodiments, the insulin and amylin are provided in a molar ratio between about 2.5:1 and 35:1 or about 5:1 and 25:1, and at least 0.5 micrograms of amylin per kilogram of the mammal per day are provided.

Preferably the composition is provided in a form which allows delayed release of both the insulin and amylin in a constant molar ratio, or in a form suitable for parenteral administration.

The compositions of the invention may be administered to mammals having a reduced ability to produce insulin compared to a normal mammal. Most preferably, the mammal is a human, e.g., suffering from diabetes mellitus. Prior to administration the level of insulin and amylin may be determined in the mammal, and then an amount of an insulin and an amylin may be administered which will provide a molar ratio of insulin and amylin in the serum of the animal between about 2.5:1 and 35:1.

The level of insulin and amylin in the mammal may be determined by any desired means, many examples of which exist in the published literature. In addition, amylin activity can be assayed as described by Cooper and Young, U.S. Serial No. 07/666,512, entitled "Amylin Activity Assays", filed March 8, 1991, assigned to the same assignee as the present application.

In a related aspect, the invention provides use as defined above wherein the composition comprising insulin and amylin provides a circulating plasma level of amylin in a mammal that is about 3 to 6 percent that of insulin.

One of the main advantages of the present invention may be the reduction of the adverse effects of insulin overdose so long as the correct ratio of amylin is also provided with that insulin. At present, in order to avoid the acutely unpleasant effects of hypoglycemia, patients characteristically tend to hold back on their insulin therapy, preferring to live with higher levels of plasma glucose which do not present any immediately discernible pathologies. However, the resulting chronic hyperglycemia leads to vascular defects, insulin resistance, and abnormal carbohydrate metabolism. With the present invention, such diabetics need no longer be resigned to this chronic state of hyperglycemia. Thus, the optimum amylin/insulin combination of this invention aids in overcoming long-term complications normally associated with insulin therapy.

The amylin/insulin formulations of this invention provides patients who inject their own insulin with an improved formulation that reduces the occurrence of low plasma glucose levels. These formulations impede glucose disposal into skeletal muscle when plasma glucose levels are depressed, stimulate hepatic glycogen synthesis to ensure an effective counterregulatory response to hypoglycemia, and permit essentially normal insulin control of hyperglycemia.

The blended insulin-amylin formulations place a limit on the rate of glucose disposal into skeletal muscle, which decreases the risk of hypoglycemia associated with aggressive hormone therapy. Additionally, the amylin will enhance or restore fatty acid synthesis from lactate and provide the patients a more normal body fat distribution.

Other advantages of this invention to diabetics include lower average plasma glucose levels; reduced long term complications; relaxation of monitoring needs; less need for rigorous definition of insulin dosage; greater freedom to vary size and timing of hormone therapy, and the extent and timing of any rigorous activity; and a better sense of well being.

As with insulin alone, the above insulin/amylin formulations can be tailored by standard techniques to the specific physiological requirements of each patient. Other features and advantages of the invention will be apparent from the following description of the preferred embodiments thereof, and from the claims.

Description of the Preferred Embodiments

The drawings will first briefly be described.

4

<u>Drawings</u>

Fig. 1 is a representation, using standard single letter nomenclature to represent amino acids, of the amino acid sequence of amylin and related polypeptides;

Fig. 2 is a graphical representation of insulin dose response in rat soleus muscle;

Fig. 3 is a graphical representation of a family of insulin dose response curves with different fixed concentrations of amylin;

Fig. 4 is a graphical representation of a family of amylin dose response curves with different fixed concentrations of insulin;

Fig. 5 is a graphical representation showing an insulin/amylin dose response surface, the lines labelled 1, 14, and 100 representing the predicted responses for insulin/amylin mixtures where amylin is at 1%, 14% and 100% of the molar concentration of insulin, respectively.

Fig. 6 is a graphical representation showing response to increasing insulin and amylin concentration in a fixed ratio of 7 to 1; the upper line shows the sigmoid dose response for insulin alone;

Fig. 7 is a graphical representation showing amylin dose response curves utilizing three different methods for measuring experimental amylin concentrations; and

Fig. 8 is a graphical representation of liver glycogen levels in rats, showing the effect of insulin and insulin/amylin combinations in restoring glycogen levels in streptozotocin-treated diabetic rats.

<u>Detailed Description</u>

The following experiments demonstrate the interaction of amylin and insulin in an <u>in vitro</u> and an <u>in vivo</u> test format. These experiments illustrate the surprising nature of interaction of the two hormones in regulation of glycogen metabolism.

As described in more detail below, at low to moderate rates of insulin secretion, the range for most physiologic situations, the response to insulin in the presence of amylin in not much altered. The slope of the insulin dose response is reduced as amylin concentrations begin to reach levels that significantly depress the insulin response. The maximum response achievable is considerably lower than the maximum that could be elicited by insulin alone. As insulin and amylin concentrations rise further, into ranges not normally expected to occur, the insulin response actually declines as increasing amylin action further depresses the functional response to insulin.

<u>Example 1: Isolated Soleus Muscle of Rat</u>

Soleus assay, dissection

Male Harlan Sprague Dawley rats of approximately 200 g mass were used in order to maintain soleus muscle mass less than 40 mg. The animals were fasted for 4 hours prior to sacrifice by decapitation. The skin was stripped from the lower limb which was then pinned out on cork board. The <u>tendo achilles</u> was cut just above <u>os calcis</u> and <u>m. gastrocnemius</u> reflected out from the posterior aspect of the tibia. <u>M. soleus</u>, a small 15-20 mm long, 0.5 mm thick flat muscle on the bone surface of <u>m. gastrocnemius</u> was then stripped clear and the perimysium cleaned off using fine scissors and forceps. <u>M. soleus</u> was then split into equal parts using a blade passed antero-posteriorly through the belly of the muscle to obtain a total of 4 muscle strips from each animal. After dissecting the muscle from the animal, it was kept for a short period in physiological saline. The muscle was not held under tension, as reported in other methodologies since in our hands this had no demonstrable effect on the insulin-sensitivity of radioglucose incorporation into glycogen.

<u>Incubation</u>

Muscles were added to 50 mL Erlenmeyer flasks containing 10 mL of a pregassed Krebs-Ringer bicarbonate buffer containing (mM) NaCl 118.5; KCl 5.94; $CaCl_2$ 2.54; $MgSO_4$ 1.19; $KH_2PO_4$ 1.19; $NaHCO_3$ 25; glucose 5.5; and recombinant human insulin (Humulin-R, Eli Lilly, IN) and either synthetic rat (Bachem, Torrance, CA. Lot# ZG485) or synthetic human amylin (Bachem, Torrance, CA. Lot# ZH144) as detailed below. Methods used to determine the nature and purity of rat and human amylin included quantitative amino acid analysis, gas phase amino acid sequencing, reverse phase HPLC and fast-atom-bombardment mass spectrometry. By the latter two methods, purity was 98.4 and 97.9% respectively. However, application of these methods failed to detect 1 atom of mercury per molecule of rat amylin, which was subsequently detected by electrospray ionization mass spectrometry and atomic absorption spectroscopy. Subsequent analysis of the method of synthesis of the rat amylin suggests that this atom is interposed between the sulfur atoms of cysteine residues 2 and 7, and therefore forms part of the ring structure near the amino-terminus. Incorporation of mercury into rat amylin may be a feature of many commercially available batches that have already been used in published studies. Since the peptide was bicactive, its action in muscle was expected to at least qualitatively mimic that of true rat amylin. The mercuric method of cyclization was not used in the synthesis of human amylin, which is found by electrospray ionization mass spectrometry and atomic absorption spectroscopy to be mercury-free.

Muscles were assigned to different flasks so that the four muscle pieces from each animal were evenly distributed among the different assay conditions. The incubation media were gassed by gently blowing carbogen (95% $O_2$, 5% $CO_2$) over the surface while it was continuously agitated at 37°C in an oscillating water bath. pH of gassed media at 37°C was verified as being between 7.1 and 7.4. After a half-hour preincubation, 0.5 μCi of [U-$^{14}$C]-glucose was added to each flask for a further 60 minutes. Each muscle piece was then rapidly removed, trimmed of tendons, blotted, frozen in liquid $N_2$, weighed and then stored at 20°C for subsequent determination of $^{14}$C-glycogen. The incubation medium was also frozen for subsequent analysis. The concentration of human amylin present in the medium at the end of the incubation was determined by radioimmunoassay in surfactant-containing buffer. For both human and rat amylin, the actual mass added to the incubation medium was determined by quantitative amino-acid analysis.

### $^{14}$C-Glycogen Determination

Each frozen muscle specimen was placed in a vial with 1 mL 60% potassium hydroxide (wt/vol) and digested at 70°C for 45 minutes under intermittent vigorous agitation. Dissolved glycogen was precipitated onto the walls of the vial by addition of 3 mL absolute ethanol and overnight cooling at -20°C. After centrifugation for 30 minutes at 2000 x g, the supernatant was gently aspirated, the glycogen was again washed with ethanol and centrifuged, the ethanol aspirated and the precipitate dried under vacuum. It was important to evaporate all ethanol to avoid quenching during scintillation counting. The remaining glycogen was redissolved in 1 mL water and 4 mL scintillation fluid and counted for $^{14}$C.

### Numerical Methods

A rate of glucose incorporation into glycogen (expressed in μmol/hr/g wet tissue) was obtained from the specific activity of $^{14}$C-glucose in the 5.5 mM glucose of the incubation medium, and the total $^{14}$C counts remaining in the glycogen extracted from each muscle. Dose response curves were fitted to a 4-parameter logistic model using a least-squares iterative routine (ALLFIT, v2.7, NIH, MD) to derive $EC_{50}$'s. Since $EC_{50}$ is log-normally distributed, it is expressed $\pm$ standard error of the logarithm. Pairwise comparisons were performed using t-test based routines of SYSTAT.

### Insulin dose response

Insulin dose response curves were obtained by incubation in media containing insulin added at final concentrations of 0, 0.071, 0.21, 0.71, 2.1, 7.1 and 71 nM[1].

### Amylin dose response

Amylin dose response curves were generated using muscles added to media containing 7.1 nM recombinant human insulin and either synthetic rat or synthetic human amylin (Bachem, Torrance, CA) added at final (nominal) concentrations of 0, 1, 3, 10, 30, 100, 300 and 1000 nM.

A supraphysiological insulin concentration was used for two reasons: (1) the precision of estimation of $EC_{50}$ was related to the magnitude of $^{14}$C incorporation into glycogen, so greater amylin suppression of the insulin response was possible with near-maximal insulin stimulation; and (2) using an insulin response near the plateau (asymptotic part) of the dose response curve in contrast to the 50% response obtained at the often-used insulin concentration of 710 pM minimized variation in response due to variation in the bathing insulin concentration or individual muscle insulin sensitivities. The effect of using higher insulin concentrations is seen in the reduced standard error of the amylin dose response $ED_{50}$'s detailed in Table 1.

Each assay also contained internal positive controls consisting of a single batch (Bachem Lot# 2G485) of archived rat amylin, lyophilized and stored at -20°C.

Referring to Fig. 9, data pooled from 5 amylin dose response assays performed on a single batch of human amylin in the presence of 7.1nM insulin are shown. Curve A was fitted to the data obtained when amylin concentration

[1] Conversion used, 1μU/mL=7.1pM.

is derived from mass initially weighed out and diluted. Curve B was fitted to data obtained when amylin concentration is derived from mass of peptide added to assay media as determined by quantitative amino-acid analysis. Curve C was fitted to data obtained when amylin concentration in the assay media is measured by radioimmunoassay. The $EC_{50}$ for curve C is 438pM $\pm$ 0.17 log units (SE).

Insulin/amylin response matrix

The insulin/amylin dose response matrix was obtained by repeating the insulin dose response analyses with amylin added to nominal final concentrations of 0, 3, 10, 30, 100 and 300 nM, thereby generating a 6x6 table with quadruplicates at each combination of insulin and amylin concentrations.

Insulin:amylin fixed ratio dose response

Muscles were incubated in media containing synthetic rat amylin and recombinant human insulin added in a 1:7.1 molar ratio.

Insulin and amylin dose responses

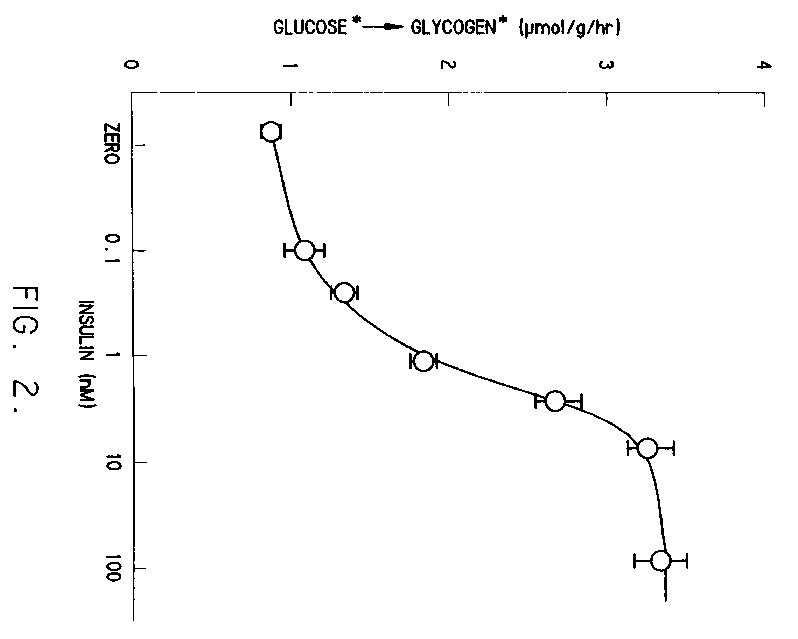

Data pooled from 7 insulin dose response assays performed in the absence of amylin are shown in Fig 2. There was a 3.7-fold increment in rate of glycogen labelling, with an $EC_{50}$ of the response of 1.05 nM $\pm$ 0.07 log units (n=8 to 28 per point).

Combined insulin/amylin dose response

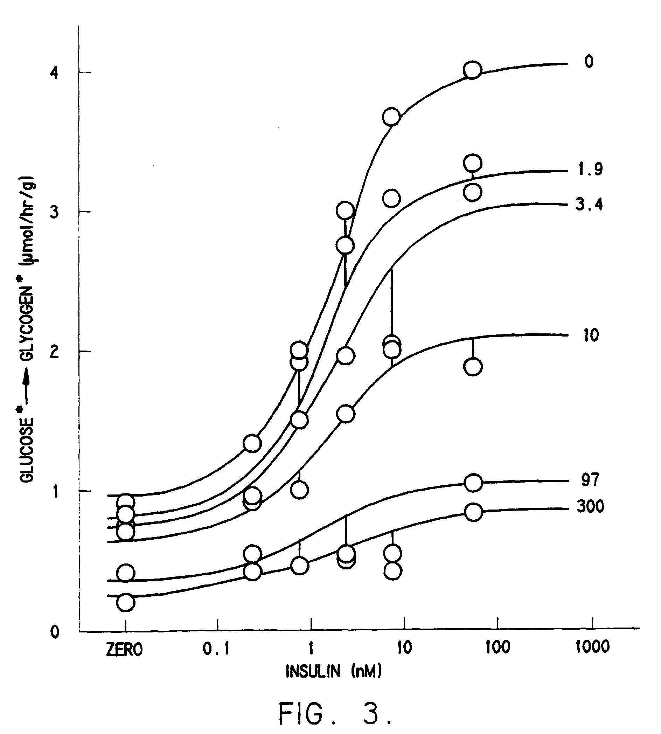

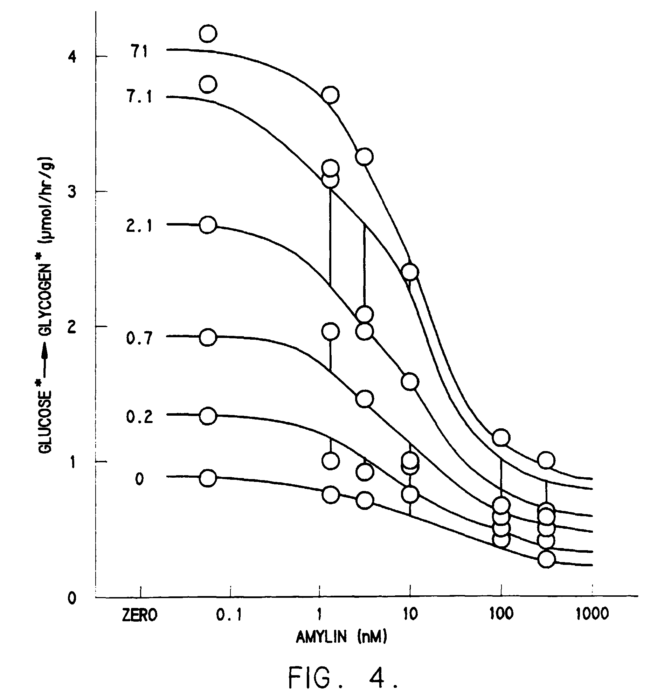

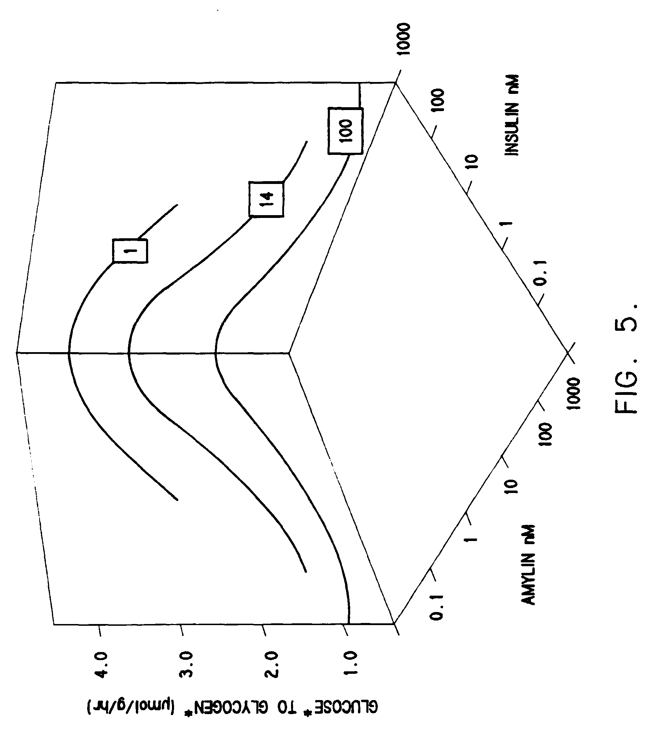

Fig 3 shows a series of insulin dose response curves obtained in the presence of different amylin concentrations as indicated. Data for the maxima and $EC_{50}$'s of the insulin responses are presented in Table 1. They indicate that the $EC_{50}$ of the insulin response was not progressively right-shifted with increasing amylin concentrations. Fig 4 shows amylin dose response curves obtained with different insulin concentrations, as indicated. Amylin $EC_{50}$'s and maximal responses, presented in Table 1, similarly indicate that the $EC_{50}$ of the amylin response was independent of the prevailing insulin concentration. In other words, amylin could suppress labelling of glycogen at all insulin concentrations. Fig 5 combines the data from the insulin and amylin dose responses into a single fitted surface. The surface was generated as the product of fractional responses to insulin and amylin, using means of parameters for slope and $EC_{50}$ for the insulin and amylin transects, and plotted on log/log/linear axes.

The equation for the surface is

$$\left| \frac{A_{ins} - D_{ins}}{1 + \left( \frac{Insulin}{C_{ins}} \right) B_{ins}} + D_{ins} \right| \; X \; \left| \frac{A_{amy} - D_{amy}}{1 + \left( \frac{Amylin}{C_{amy}} \right) B_{amy}} + D_{amy} \right| \; X \; F + K$$

The equation thus has the general form... Response = [Insulin factor] x [Amylin factor] x F + K  where

| $A_{ins}$ = 1 | $A_{amy}$ = 1 | (maximal responses) |
|---|---|---|
| $B_{ins}$ = -0.889 | $B_{amy}$ = 0.835 | (slope factors) |
| $C_{ins}$ = 1.42 nM | $C_{amy}$ = 9.0 nm | ($EC_{50}$s) |
| $D_{ins}$ = 0.18 | $D_{amy}$ = 0.15 | (basal responses) |
| F (response factor) | = 4.0 µmol/hr/g wet muscle | |
| K (constant) | = 0.14 | |
| Insulin and Amylin concentrations are in nM | | |

Since the baseplane of the graph is defined by log [insulin] and log [amylin] axes, combinations of insulin and amylin in any given ratio are represented as straight lines on this plane. Lines describing amylin:insulin ratios of 0.01:1, 0.14:1 and 1:1 are projected up onto the surface (labelled 0.01, 0.14 and 1 respectively).

Insulin:amylin fixed ratio dose response

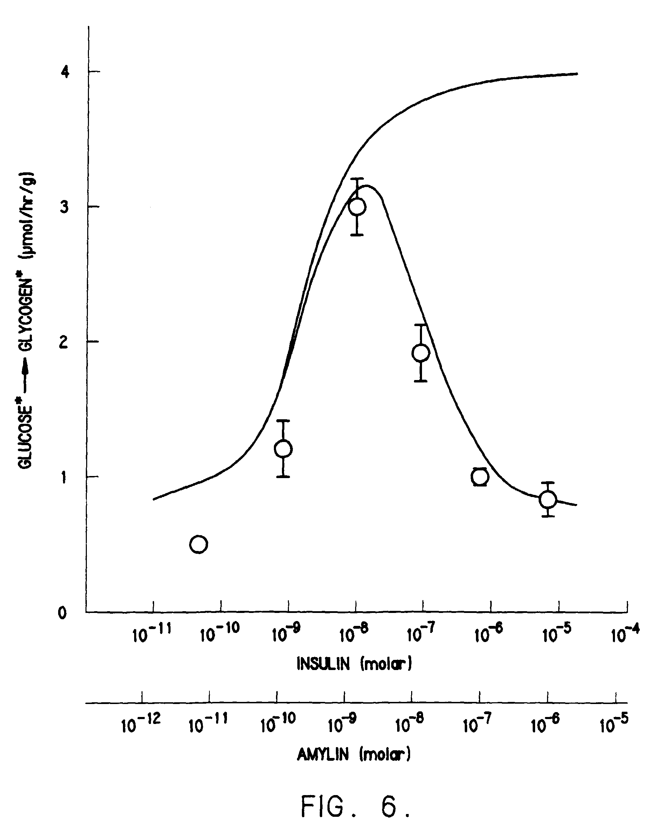

The experimentally determined response to increasing concentrations of a fixed 0.14:1 ratio of amylin:insulin are plotted as open circles in Fig 6. The bell-shaped function generated from Equation 1, using [amylin]= [insulin] x 0.14, and which corresponds to the trajectory labelled "0.14" in Fig 5, is shown as a continuous line. Also plotted is the sigmoid dose response predicted for insulin in the absence of amylin. The data conform to the bell-shaped profile predicted for non-competitive functional antagonism rather than to a shifted sigmoid response curve as would be predicted for competitive antagonism.

The finding that the $EC_{50}$'s for amylin and insulin action on soleus muscle are not altered by increasing concentrations of the other hormone are consistent with biochemical and pharmacological data indicating that they act via separate receptors rather than by competing at a common receptor. Amylin behaves as a noncompetitive, functional antagonist to insulin. That is, amylin reduces the magnitude of the insulin response without affecting insulin potency. Critically important for analyzing the role of amylin is the finding that it causes insurmountable inhibition of insulin action.

Amylin's biological activity was originally described as an "antagonism" of the muscle response to insulin. Some may therefore have considered that amylin and insulin represent self-canceling signals, and that if these hormones are secreted in a constant ratio, the effects of any increase in plasma amylin would be nullified by the proportionate increase in insulin. The "constant ratio" slices through the response surface (Fig 4) instead predict that with any fixed ratios of insulin and amylin, suppression by amylin of the insulin response will eventually supervene as the concentrations of both hormones increase. This prediction was confirmed by the experiments shown in Fig 6. If amylin and insulin were cosecreted in a fixed ratio, the bell-shaped trajectory of Fig 6 would represent the response of muscle to increasing secretion from the 8-cell. The peak height, the position and shape of the insulin:amylin bell-shaped curve are functions of the muscle sensitivities to insulin and amylin, and their concentration ratio.

Table 1

| Insulin Dose Response | | |
|---|---|---|
| [amylin] nM (nominal) | Maximal response µmol/g wet tissue/hr | $EC_{50}$ nm |
| | Mean | Mean $\pm$ log SE |
| 0 | 4.09 | 1.16 $\pm$ 0.10 |
| 3 | 3.45 | 0.78 $\pm$ 0.12 |
| 10 | 3.12 | 1.52 $\pm$ 0.15 |
| 30 | 2.13 | 1.33 $\pm$ 0.20 |
| 100 | 1.15 | undetermined |
| 300 | 0.93 | undetermined |
| Amylin Dose Response | | |
| [insulin] nM (nominal) | Maximal response µmol/g wet tissue/hr | $EC_{50}$ nm |
| | Mean $\pm$ SEM | Mean $\pm$ log SE |
| 0 | 0.85 $\pm$ 0.17 | 12.15 $\pm$ 0.94 |
| 0.21 | 1.26 $\pm$ 0.18 | 10.27 $\pm$ 0.48 |
| 0.71 | 2.11 $\pm$ 0.18 | 8.24 $\pm$ 0.24 |
| 2.1 | 2.94 $\pm$ 0.18 | 10.45 $\pm$ 0.16 |
| 7.1 | 3.56 $\pm$ 0.19 | 8.69 $\pm$ 0.13 |
| 71 | 4.07 $\pm$ 0.18 | 11.93 $\pm$ 0.11 |

Example 2: Daily Amylin replacement reverses hepatic glycogen depletion in insulin-treated streptozotocin diabetic rats.

In streptozotocin-diabetic rats treated with insulin replacement, liver glycogen is some 35% depleted. Five consecutive daily subcutaneous injections with amylin dose-dependently restored liver glycogen to normal levels. Significant increases over insulin-only therapy occurred with amylin doses of 10, 30 and 100µg/day, representing amylin: insulin ratios of 0.22, 0.75 and 2.79.

Animals

There were 68 male Harlan Sprague Dawley rats (mass 301±3g) in 8 treatment groups. Animals were housed at 22.7±0.8°C in a 12:12 hour light:dark cycle (experiments being performed during the light cycle) and fed and watered ad libitum (Diet LM-485, Teklad, Madison, WI). The rats were sacrificed for harvesting of livers 4 to 5 hours into the light cycle.

Treatment Groups

1. STZ diabetic, insulin-only treatment (n=11).

Animals were injected with streptozotocin (Sigma Chemical Company, St Louis, MO: Sigma 50130) dissolved in water in a dose of 65mg/kg into the lateral tail vein. Upon exhibiting 5% glycosuria (Chemstrip uGH, Boehringer-Mannheim, FRG), rats were commenced upon a sliding-scale daily insulin treatment regime (Humulin-Ultralente, Eli Lilly, Indianapolis, IN) aimed towards maintaining aketonuria (by Chemstrip) but 5% glycosuria. Maintaining diabetic rats in this metabolic state optimizes survival. Following one week of established diabetes, animals received once daily s.c. injections of amylin vehicle (water for injection) for 5 days given at the time of the insulin injection.

2-6 STZ diabetes, insulin+amylin treatment groups.

These animals were treated identically to those in group 1 except that the daily subcutaneous injection contained 3µg (n=5); 10µg (n=12); 30µg (n=5); 100µg (n=5) or 300µg (n=5) of rat amylin (Bachem lot# WG485[2/]). The bioactivity of the peptide used in these experiments was first verified by bioassay, using inhibition of insulin- stimulated radioglucose incorporation into glycogen in the isolated stripped rat soleus muscle. The $EC_{50}$ derived for the peptide used was 6.2nM (±0.2 log unit). The insulin dose in each of the groups of diabetic animals averaged 1.67U/animal/day. There were no other observable differences in the required management of the different groups.

7. Normal animals (n=10)

These animals were derived from the same stock and housed under the same conditions for the same time as those in groups 1-6, but were given no injections. 24 hours after the fifth daily injection of amylin or water (or an equivalent time after admission to the vivarium in the case of group 7 rats), the non-fasted rats were killed by decapitation and the livers immediately removed and frozen in liquid $N_2$, weighed and stored for subsequent determination of glycogen concentration.

8. STZ-diabetic animals, no treatment (n=10)

These animals were made diabetic as were groups 1-6, but received neither insulin nor amylin.

Glycogen determination

Whole livers were powdered while frozen. Approximately 200mg of powder was further homogenized in 1.0mL of 0.6M perchloric acid to denature enzymes. 200µL of the homogenate was neutralized with 0.5 volumes of 1.0M $KHCO_3$ in either of two acetate buffer solutions, one containing 2.0mL of 200mM acetate (pH4.8), the other containing the same but with 18.5 U/mL of amyloglucosidase (EC5 3.2.1.3, from Aspergillus niger, Sigma A3423, Sigma Chemical Company, St Louis, MO) added. Following at least 20 minutes incubation at 23°C, the supernatants were assayed for glucose in an analyzer using D-glucose oxidase immobilized enzyme chemistry (Analyzer model 2300-

[2/] This batch of rat amylin has subsequently been found to contain a mercury contaminant, believed to be interposed between the sulphur atoms of the 2-cys 7-cys disulphide bridge.

STAT, YSI, Yellow Springs, OH). Purified rabbit liver glycogen (Sigma G8876) used as a standard indicated 98±4% recovery of dissolved glycogen and linearity within the range of observed liver glycogen concentrations (r=0.9994). All reagents were of analytical grade or better.

Numerical methods

Statistical comparisons were by Student's $t$-test routines contained in the SYSTAT system (Systat, Evanston, IL). All results are reported as means ± standard error of the mean.

Liver glycogen

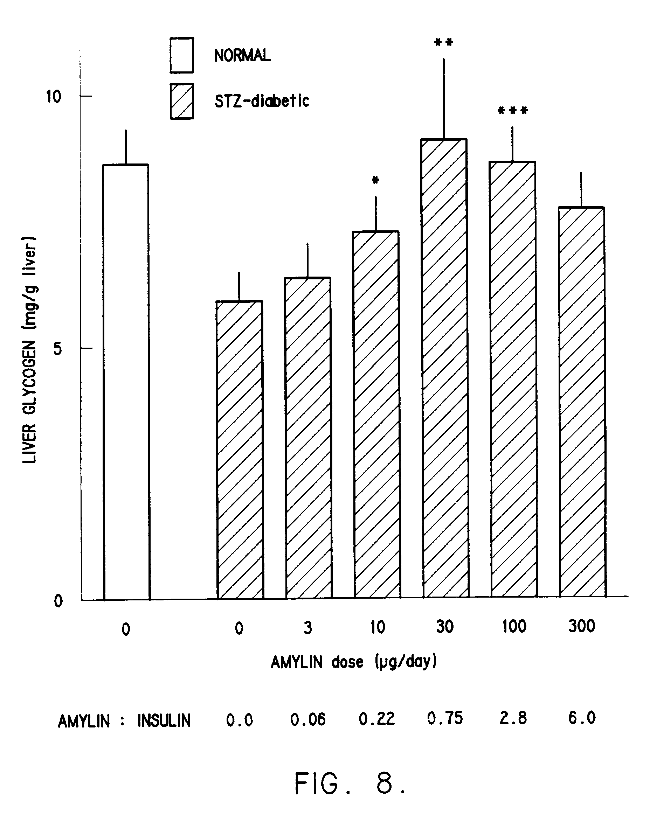

Liver glycogen content was measured in rats with free access to food up to the time of sacrifice (fasting rapidly depletes liver glycogen, such that the levels after 18-hours starvation are typically only 2-5% of those observed in the fed state). Glycogen contents for the 8 groups of animals are shown in Fig 8. STZ diabetic animals on no therapy showed a 67% decrease in liver glycogen concentration compared to normal rats (2.86 $\underline{vs}$ 8.6 mg/g, $\underline{P}$<0.001). STZ diabetic animals receiving insulin replacement had a 35% decrease in liver glycogen compared to normal rats (5.6 $\underline{vs}$ 8.6 mg/g, $\underline{P}$<0.01). In insulin-treated STZ diabetic rats supplemented with daily amylin, there was a dose-dependent increase in liver glycogen concentration above that in rats replaced with insulin alone ($\underline{P}$<0.05, 10µg/day; $\underline{P}$<0.02, 30µg/day; $\underline{P}$<0.01, 100µg/day). The liver glycogen concentration in animals receiving 10, 30 and 100µg amylin per day was not significantly different from that in normal animals ($\underline{P}$<0.3), although the mean values showed a dose-dependent increase with an apparent peak at 30µg/day. The lowest dose examined, 3µg/day did not measurably increase glycogen levels above those treated with insulin alone. Interestingly, the highest dose of amylin tested, 300µg/day, also did not measurably restore liver glycogen towards normal, giving an apparently biphasic dose-response.

The results show that combined replacement of amylin and insulin can restore normal levels of liver glycogen in STZ-diabetic rats; full restoration is not achieved with insulin alone. This physiological response is caused by a once-daily subcutaneous injection in a molar ratio of amylin to insulin close to that thought to occur naturally in healthy animals.

Those of ordinary skill in the art reviewing both examples above will recognize that the data indicate to one of ordinary skill in the art that administration of insulin and amylin in the indicated ratios will be an effective treatment for diabetes in humans. Such amylin and insulin can be administered in any standard manner using pharmaceutically acceptable buffers. For example, the hormones may be administered in a form which causes delayed release of the hormones within the body.

Other embodiments are within the following claims.

**Claims**

**Claims for the following Contracting States : AT, BE, CH, DE, DK, FR, GB, IT, LI, LU, MC, NL, SE**

1. A composition comprising an insulin having an *in vitro* activity of stimulating glucose incorporation into glycogen in rat soleus muscle, and an amylin having the *in vitro* activity of suppressing glucose incorporation into glycogen in rat soleus muscle, wherein said insulin and amylin are provided in a molar ratio of between 1:1 and 40:1.

2. A composition according to claim 1, wherein said insulin and amylin are provided at a molar ratio of between 2.5:1 and 25:1.

3. A composition according to claim 1, wherein said insulin and amylin are provided at a molar ratio of between 5:1 and 10:1.

4. A composition according to any one of claims 1 to 3, wherein said composition is provided in a form suitable for parenteral administration.

5. A composition according to any one of claims 1 to 4, wherein said insulin is provided in a fast-acting form.

6. A composition according to any one of claims 1 to 5, for use in a method of treatment of the human or animal body.

7. Use of a composition comprising an insulin and an amylin wherein said insulin and amylin are provided in a molar ratio of between 1:1 and 40:1 for the manufacture of a medicament for the treatment of a mammal having a reduced ability to produce serum insulin compared to a normal mammal.

**8.** Use according to claim 7 wherein the composition is as defined in any one of claims 2 to 5.

**9.** Use according to claim 7 or 8, wherein said amylin is provided in an amount of at least 0.5 micrograms per kilogram of said mammal per day.

**10.** Use according to claim 7, 8 or 9 wherein the composition comprising insulin and amylin provides a circulating plasma level of amylin in a mammal that is about 3 to 6 percent that of insulin.

**Claims for the following Contracting States : ES, GR**

**1.** A process for the preparation of a composition comprising an insulin having an *in vitro* activity of stimulating glucose incorporation into glycogen in rat soleus muscle, and an amylin having the *in vitro* activity of suppressing glucose incorporation into glycogen in rat soleus muscle, which process comprises blending insulin and amylin in a molar ratio of between 1:1 and 40:1.

**2.** A process according to claim 1, wherein said insulin and amylin are blended at a molar ratio of between 2.5:1 and 35:1.

**3.** A process according to claim 1, wherein said insulin and amylin are blended at a molar ratio of between 5:1 and 10:1.

**4.** A process according to any one of claims 1 to 3, wherein said composition produced is in a form suitable for parenteral administration.

**5.** A process according to any one of claims 1 to 4, wherein said insulin is provided in a fast-acting form.

**6.** Use of a composition comprising an insulin and an amylin wherein said insulin and amylin are provided in a molar ratio of between 1:1 and 40:1 for the manufacture of a medicament for the treatment of a mammal having a reduced ability to produce serum insulin compared to a normal mammal.

**7.** Use according to claim 6 wherein the composition is as defined in any one of claims 2 to 5.

**8.** Use according to claim 6 or 7, wherein said amylin is provided in an amount of at least 0.5 micrograms per kilogram of said mammal per day.

**9.** Use according to claim 6, 7 or 8 wherein the composition comprising insulin and amylin provides a circulating plasma level of amylin in a mammal that is about 3 to 6 percent that of insulin.

**10.** A composition comprising an insulin having an *in vitro* activity of stimulating glucose incorporation into glycogen in rat soleus muscle, and an amylin having the *in vitro* activity of suppressing glucose incorporation into glycogen in rat soleus muscle, wherein said insulin and amylin are provided in a molar ratio of between 1:1 and 40:1.

**11.** A composition according to claim 10 which is further defined as in any one of claims 2 to 5.

**12.** A composition according to claim 10 or 11, for use in a method of treatment of the human or animal body.

**Patentansprüche**

**Patentansprüche für folgende Vertragsstaaten : AT, BE, CH, DE, DK, FR, GB, IT, LI, LU, MC, NL, SE**

**1.** Zusammensetzung, umfassend ein Insulin mit einer in vitro-Aktivität zur Stimulierung des Glucose-Einbaus in Glykogen im Ratten-Soleusmuskel, und ein Amylin mit der in vitro-Aktivität zur Unterdrückung des Glucose-Einbaus in Glykogen im Ratten-Soleusmuskel, wobei das Insulin und Amylin in einem Molverhältnis von 1:1 bis 40:1 bereitgestellt werden.

**2.** Zusammensetzung nach Anspruch 1, wobei das Insulin und Amylin in einem Molverhältnis von 2,5:1 bis 35:1 bereitgestellt werden.

**3.** Zusammensetzung nach Anspruch 1, wobei das Insulin und Amylin in einem Molverhältnis von 5:1 bis 10:1 bereitgestellt werden.

**4.** Zusammensetzung nach einem der Ansprüche 1 bis 3, wobei die Zusammensetzung in einer für die parenterale Verabreichung geeigneten Form bereitgestellt wird.

**5.** Zusammensetzung nach einem der Ansprüche 1 bis 4, wobei das Insulin in einer schnellwirkenden Form bereitgestellt wird.

**6.** Zusammensetzung nach einem der Ansprüche 1 bis 5, zur Verwendung bei einer Methode zur Behandlung des menschlichen oder tierischen Körpers.

**7.** Anwendung einer Zusammensetzung, umfassend ein Insulin und ein Amylin, wobei das Insulin und Amylin in einem Molverhältnis von 1:1 bis 40:1 bereitgestellt werden, für die Herstellung eines Medikaments zur Behandlung eines Säugers mit einer verminderten Fähigkeit zur Produktion von Serum-Insulin im Vergleich zu einem normalen Säuger.

**8.** Verwendung nach Anspruch 7, wobei die Zusammensetzung wie in einem der Ansprüche 2 bis 5 definiert ist.

**9.** Verwendung nach Anspruch 7 oder 8, wobei das Amylin in einer Menge von mindestens 0,5 Mikrogramm pro Kilogramm des Säugers pro Tag bereitgestellt wird.

**10.** Verwendung nach Anspruch 7, 8 oder 9, wobei die Zusammensetzung, umfassend Insulin und Amylin, einen zirkulierenden Plasmagehalt an Amylin in einem Säuger bereitstellt, der etwa 3 bis 6 Prozent dessen an Insulin beträgt.

**Patentansprüche für folgende Vertragsstaaten : GR, ES**

**1.** Verfahren für die Herstellung einer Zusammensetzung, umfassend ein Insulin mit einer in vitro-Aktivität zur Stimulierung des Glucose-Einbaus in Glykogen im Ratten-Soleusmuskel, und ein Amylin mit der in vitro-Aktivität zur Unterdrückung des Glucose-Einbaus in Glykogen im Ratten-Soleusmuskel, wobei das Verfahren das Mischen von Insulin und Amylin in einem Molverhältnis von 1:1 bis 40:1 umfaßt.

**2.** Verfahren nach Anspruch 1, wobei das Insulin und Amylin in einem Molverhältnis von 2,5:1 bis 35:1 gemischt werden.

**3.** Verfahren nach Anspruch 1, wobei das Insulin und Amylin in einem Molverhältnis von 5:1 bis 10:1 gemischt werden.

**4.** Verfahren nach einem der Ansprüche 1 bis 3, wobei die Zusammensetzung in einer für die parenterale Verabreichung geeigneten Form hergestellt wird.

**5.** Zusammensetzung nach einem der Ansprüche 1 bis 4, wobei das Insulin in einer schnellwirkenden Form bereitgestellt wird.

**6.** Anwendung einer Zusammensetzung, umfassend ein Insulin und ein Amylin, wobei das Insulin und Amylin in einem Molverhältnis von 1:1 bis 40:1 bereitgestellt werden, für die Herstellung eines Medikaments zur Behandlung eines Säugers mit einer verminderten Fähigkeit zur Produktion von Serum-Insulin im Vergleich zu einem normalen Säuger.

**7.** Verwendung nach Anspruch 6, wobei die Zusammensetzung wie in einem der Ansprüche 2 bis 5 definiert ist.

**8.** Verwendung nach Anspruch 6 oder 7, wobei das Amylin in einer Menge von mindestens 0,5 Mikrogramm pro Kilogramm des Säugers pro Tag bereitgestellt wird.

**9.** Verwendung nach Anspruch 6, 7 oder 8, wobei die Zusammensetzung, umfassend Insulin und Amylin, einen zirkulierenden Plasmagehalt an Amylin in einem Säuger bereitstellt, der etwa 3 bis 6 Prozent dessen an Insulin beträgt.

**10.** Zusammensetzung, umfassend ein Insulin mit einer in vitro-Aktivität zur Stimulierung des Glucose-Einbaus in Glykogen im Ratten-Soleusmuskel, und ein Amylin mit der in vitro-Aktivität zur Unterdrückung des Glucose-Einbaus in Glykogen im Ratten-Soleusmuskel, wobei das Insulin und Amylin in einem Molverhältnis von 1:1 bis 40:1 bereitgestellt werden.

**11.** Zusammensetzung nach Anspruch 10, die außerdem definiert ist wie in einem der Ansprüche 2 bis 5.

**12.** Zusammensetzung nach Anspruch 10 oder 11, zur Verwendung bei einer Methode zur Behandlung des menschlichen oder tierischen Körpers.

**Revendications**

**Revendications pour les Etats contractants suivants : AT, BE, CH, DE, DK, FR, GB, IT, LI, LU, MC, NL, SE**

**1.** Composition comprenant une insuline ayant une activité *in vitro* de stimulation de l'incorporation du glucose dans le glycogène dans le muscle soléaire chez le rat, et une amyline ayant une activité *in vitro* de suppression de l'incorporation du glucose dans le glycogène dans le muscle soléaire chez le rat, dans laquelle ladite insuline et ladite amyline sont fournies dans un rapport molaire compris entre 1:1 et 40:1.

**2.** Composition selon la revendication 1, dans laquelle ladite insuline et ladite amyline sont fournies dans un rapport molaire compris entre 2,5:1 et 35:1.

**3.** Composition selon la revendication 1, dans laquelle ladite insuline et ladite amyline sont fournies dans un rapport molaire compris entre 5:1 et 10:1.

**4.** Composition selon l'une quelconque des revendications 1 à 3, dans laquelle ladite composition est fournie sous une forme convenant pour une administration parentérale.

**5.** Composition selon l'une quelconque des revendications 1 à 4, dans laquelle ladite insuline est fournie sous une forme à action rapide.

**6.** Composition selon l'une quelconque des revendications 1 à 5, destinée à être utilisée dans un procédé de traitement du corps humain ou animal.

**7.** Utilisation d'une composition comprenant une insuline et une amyline dans laquelle ladite insuline et ladite amyline sont fournies dans un rapport molaire compris entre 1:1 et 40:1 pour la préparation d'un médicament destiné au traitement d'un mammifère ayant une capacité réduite pour produire de l'insuline sérique par rapport à un mammifère normal.

**8.** Utilisation selon la revendication 7 dans laquelle la composition est telle que définie selon l'une quelconque des revendications 2 à 5.

**9.** Utilisation selon la revendication 7 ou 8, dans laquelle ladite amyline est fournie en une quantité journalière d'au moins 0,5 μg par kg dudit mammifère.

**10.** Utilisation selon la revendication 7, 8 ou 9 dans laquelle la composition comprenant de l'insuline et de l'amyline procure un taux d'amyline en circulation dans le plasma chez un mammifère qui est d'environ de 3 à 6 % celui de l'insuline.

**Revendications pour les Etats contractants suivants : GR, ES**

**1.** Procédé de préparation d'une composition comprenant une insuline ayant une activité *in vitro* de stimulation de l'incorporation du glucose dans le glycogène dans le muscle soléaire chez le rat, et une amyline ayant une activité *in vitro* de suppression de l'incorporation du glucose dans le glycogène dans le muscle soléaire chez le rat, ce procédé comprenant le mélange de l'insuline et de l'amyline dans un rapport molaire compris entre 1:1 et 40:1.

**2.** Procédé selon la revendication 1, dans lequel ladite insuline et ladite amyline sont mélangées dans un rapport molaire compris entre 2,5:1 et 35:1.

3. Procédé selon la revendication 1, dans lequel ladite insuline et ladite amyline sont mélangés dans un rapport molaire compris entre 5:1 et 10:1.

4. Procédé selon l'une quelconque des revendications 1 à 3, dans lequel ladite composition est obtenue sous une forme convenant pour une administration parentérale.

5. Procédé selon l'une quelconque des revendications 1 à 4, dans lequel ladite insuline est fournie sous une forme à action rapide.

6. Utilisation d'une composition comprenant une insuline et une amyline dans laquelle ladite insuline et ladite amyline sont fournies dans un rapport molaire compris entre 1:1 et 40:1 pour la préparation d'un médicament destiné au traitement d'un mammifère ayant une capacité réduite pour produire de l'insuline sérique par rapport à un mammifère normal.

7. Utilisation selon la revendication 6 dans laquelle la composition est telle que définie selon l'une quelconque des revendications 2 à 5.

8. Utilisation selon la revendication 6 ou 7, dans laquelle ladite amyline est fournie en une quantité journalière d'au moins 0,5 µg par kg dudit mammifère.

9. Utilisation selon la revendication 6, 7 ou 8 dans laquelle la composition comprenant l'insuline et l'amyline procure un taux d'amyline en circulation dans le plasma chez un mammifère qui est d'environ de 3 à 6 % celui de l'insuline.

10. Composition comprenant une insuline ayant une activité *in vitro* de stimulation de l'incorporation de glucose dans le glycogène dans le muscle soléaire chez le rat, et une amyline ayant une activité *in vitro* de suppression de l'incorporation du glucose dans le glycogène dans le muscle soléaire chez le rat, dans laquelle ladite insuline et ladite amyline sont fournies dans un rapport molaire compris entre 1:1 et 40:1.

11. Composition selon la revendication 10 qui est en outre définie telle que selon l'une quelconque des revendications 2 à 5.

12. Composition selon la revendication 10 ou 11, utilisable dans une méthode de traitement du corps humain ou animal.

INTER-PEPTIDE (HUMANS)

```
AMYLIN    K C N T A T C A T Q R L A N F L V H S S N N F G A I L S S T N V G S N T Y — NH2
CGRP-1    A - D - - - - V - H - - - G L - S R - G G V V K N N F V P - - - - - K A F — NH2
CGRP-2    A - - - - - - V - H - - - G L - S R - G G N V K S N F V P - - - - - K A F — NH2
```

INTER-PEPTIDE (HUMANS)

```
HUMAN       K C N T A T C A T Q R L A N F L V H S S N N F G A I L S S T N V G S N T Y — NH2
BABOON      - - - - - - - - - - - - - - - - - - - - - - - - - - - - - - - - - - - — NH2
MONKEY      - - - - - - - - - - - - - - - - - R - - - - - - T - - - - - - - - D - - - NH2
CAT         - - - - - - - - - - - - - - - - I R - - - - L - - - - - P - - - - - - - - — NH2
DOG         - - - - - - - - - - - - - - - - R T - - - L - - - - - P - - - - - - - - - — NH2
RAT         - - - - - - - - - - - - - - - - R - - - - L - P V - P P - - - - - - - - — NH2
MOUSE       - - - - - - - - - - - - - - - - R - - - - L - P V - P P - - - - - - - - — NH2
HAMSTER     - - - - - - - - - - - - - - - - - N - - L - P V - - P - - - - - - - - — NH2
GUINEA PIG  - - - - - - - - - - - - T - - - - R - - H - L - - A - L P - D - - - - - - - — NH2
DEGU        - - - - - - - - - - - - T - - - - R - - H - L - - A - P P - K - - - - - - — NH2
```

FIG. 1.

FIG. 2.

FIG. 3.

FIG. 4.

FIG. 5.

EP 0 586 592 B1

FIG. 6.

FIG. 7.

FIG. 8.